# EUROPEAN PATENT SPECIFICATION

(11) **EP 4 217 706 B1**
(45) Date of publication and mention of the grant of the patent: **08.04.2026**
(21) Application number: 21778107.9
(22) Date of filing: 17.09.2021
(51) Int. Cl.: G01N 1/24, G01N 1/08, G01N 1/22, A61B 10/02, G01N 1/02, G01N 1/40

(54) **METHOD, TOOL AND APPARATUS FOR DISSECTING AND TRANSFERRING BIOLOGICAL MATERIAL**
VERFAHREN, WERKZEUG UND VORRICHTUNG ZUM SCHNEIDEN UND ZUM TRANSFER VON BIOLOGISCHEM MATERIAL
PROCÉDÉ, OUTIL ET APPAREIL DE DISSECTION ET DE TRANSFERT DE MATÉRIAU BIOLOGIQUE

(30) Priority: 22.09.2020 EP 20197369; 11.06.2021 EP 21178992
(43) Date of publication of application: 02.08.2023
(73) Proprietor: Xyall B.V., 5633 AJ Eindhoven (NL)
(72) Inventor: THIJSSEN, Edwin Johannes Richardus Wilhelmus, 5441 XB Oeffelt (NL); HAAZEN, Nicole Catharina Elisabeth, 5685 JW Best (NL); LEMBRECHTS, Thomas Patrick Anne-Lise, 5617 AK Eindhoven (NL); VIEHMANN, Kelsey, 6003 AD Weert (NL); WIMBERGER-FRIEDL, Reinhold, 5633 AJ Eindhoven (NL)
(74) Representative: DeltaPatents B.V.
(86) International application number: PCT/EP2021/075625
(87) International publication number: WO 2022/063695

(56) References cited:
- EP-A1- 2 423 661
- DE-A1- 10 011 235
- DE-A1- 10 011 235
- DE-A1- 19 917 855
- DE-A1- 19 917 855
- US-A1- 2004 142 488
- US-A1- 2004 142 488
- US-A1- 2011 061 476
- US-A1- 2013 118 017
- US-A1- 2013 118 017
- US-A1- 2014 329 269
- US-A1- 2014 329 269
- US-A1- 2019 390 252
- US-A1- 2019 390 252
- US-A1- 2020 038 001

## Description

### FIELD OF THE INVENTION

The invention relates to an automated method of dissecting biological material disposed on a planar substrate, such as a glass slide, and transferring the dissected material to a receptacle for further analysis. The invention further relates to an associated apparatus and to a disposable tool for use in the apparatus.

### BACKGROUND ART

To perform a molecular analysis of a tumor, for the purposes of oncology diagnostics, a certain amount and concentration of tumor cells must be present in the sample to be analyzed. Tumor tissue is heterogenous and contains other tissue and cell types. Therefore, a region of interest (ROI) is typically defined as the sample to be dissected from a thin section of tissue disposed on a microscope slide. Manual methods of dissection are most common, in which a lab technician scrapes material from the ROI using e.g. a scalpel blade and transfers the scraped material into a collection tube. This requires a great deal of skill and even when performed by a highly skilled technician, there is no guarantee that only material from the ROI will end up in the collection tube.

An enhanced manual method of tissue removal and collection is disclosed in US 2020038001. The method employs suction to remove tissue that has been manually scraped off a slide. The employed dissection tool has three main elements: a shaft part, one end of which can be coupled to a suction source; a cap incorporating a blade at one end and a filter column that is releasably secured to the shaft part via the cap. The filter column comprises a filter member which catches dissected material that is suctioned into the tool through the cap. After dissection, the filter column is removed from the tool and e.g. placed in a microcentrifuge tube where the filter member is submerged in a solution for dissolving the dissected tissue.

This method has the advantage of reducing the risk of contamination of tissue samples during the transfer step, but the need for a tool that can be dismantled makes the disclosed method and tool unsuitable for use in an automated device.

An example of an automated tissue dissection system employing vacuum suction is disclosed in US 2019/0390252. In one described embodiment, the tool comprises a rotationally mounted milling tip at the tool orifice, and filter element that spans an internal passageway of the tool, whereby disrupted material is sucked into the tool body and collected at the filter element. It is suggested that pressurized air can be used to force the collected material into a suitable container.

A further example of an automated device for extracting material from a biological sample via milling is disclosed in US 10876933. The device comprises a head assembly and a stage that can accommodate and collect samples from several tissue slides and enables automated filling and uploading of several milling tips into a plurality of corresponding sample collection vials. During dissection, the head assembly causes the milling tip to rotate while withdrawing a plunger which simultaneously dispenses buffer solution to the blade part of the milling tool, dissects the tissue and aspirates the buffer solution and dissected tissue into a designated collection vial by depressing the plunger. The used milling tip can then be reloaded into a holder or discarded to avoid cross-contamination.

It is also known to use laser capture microdissection (LCM) to isolate a concentrated population of individual cells or precise anatomical regions of tissue from tissue sections on a microscope slide. A method and device for transferring a microscopic, isolated sample, particularly a membrane-supported micro-dissected specimen, from an object table to an analysis arrangement, is disclosed in US 8573073. The device is equipped with nano-suction means and comprises a suction tube with a terminal membrane and a vacuum/overpressure unit coupled to the suction tube for sucking or blowing the sample onto or from the terminal membrane. The device and method of US 8573073 have the disadvantage that two different tools are required for dissection and transfer.

There is still room for improvement in terms of defining a more straightforward method, tool and apparatus for dissection and transfer of biological material with minimal contamination that allows the material to be both collected and transferred for analysis in an automated manner, using the same tool.

### SUMMARY OF THE INVENTION

In one aspect, the invention relates to an automated method of dissecting and transferring biological material from a sample disposed on a planar substrate, such as a glass slide, into a collection tube, using a dissection tool in accordance with claim 1, which has an internal passageway extending between first and second ends of the tool and an air-permeable filter element that spans the internal passageway. The tool first end is provided with an orifice and a scraping blade arranged at the orifice. The method comprises steps of:
a) physically detaching the biological material from the planar substrate using the scraping blade of the dissection tool;
b) during step a), generating an underpressure at the second end of the dissection tool, to create an uplifting airstream at the tool orifice, which suctions detached material into the internal passageway, where it is held at an underside of the filter element;
c) arranging the collection tube around the orifice, so as to be in sealing contact with a connection interface on the dissection tool; and
d) generating an overpressure at the tool second end, so as to create an airstream and pressure pulse that ejects the biological material held at the underside of the filter element, transferring the material into the collection tube.

The method of the invention enables a "dry" method of transferring the biological material from the glass slide straight to the collection tube, thereby minimizing the risk of contamination. Furthermore, a single tool can be used to dissect, collect and transfer the biological material, which not only simplifies the construction of the equipment that is needed to implement the method in an automated manner, but also further reduces the risk of contamination. The method does not utilize any liquids which would dilute the biological material and impact the methods and quality of the molecular analysis of the collected biological material.

The step of detachment comprises bringing the scraping blade into contact with the planar substrate and moving the dissection tool relative thereto. For simplicity, the planar substrate will be referred to hereafter as a glass slide, although as will be understood, any suitable type of substrate on which biological samples are disposed may be used in the method of the invention.

Advantageously, the method comprises controlling the relative positions of the tool and the glass slide such that the scraping blade engages with the biological material only in a region of interest. This optimises the amount and purity of the material that is gathered for analysis.

The step of generating an underpressure suitably comprises connecting the internal passageway of the dissection tool, at the tool second end, to a vacuum generator and the step of generating an overpressure comprises connecting the internal passageway to a pressure reservoir.

Preferably, the internal passageway of the dissection tool is selectively connectable to the vacuum generator or the pressure reservoir via a valve and the method comprises switching the valve from a first position in which the internal passageway is in fluid communication with the vacuum generator to a second position in which the internal passageway is in fluid communication with the pressure reservoir.

The pressure differential between the pressure reservoir and the sealed collection tube creates the pressure pulse that ejects the biological material from the filter element into the collection tube.

In a preferred embodiment, the method comprises a further step of using the vacuum generator to at least partially evacuate the collection tube of air before the tool second end is connected to the pressure reservoir. The advantage of evacuating the collection tube is that this increases the pressure differential between the sealed collection tube and the pressure reservoir. A further advantage of evacuating the collection tube is that the atmosphere may serve as the pressure reservoir and the step of generating an overpressure may simply comprise exposing the evacuated tube to atmospheric pressure.

Preferably, the pressure reservoir supplies a pressure higher than atmospheric pressure, e.g. 200 - 400 kPa, to ensure that the generated pressure pulse is sufficient to eject the biological material, which may be sticky in nature and adhere to the underside of the filter element. The sealing contact between the collection tube and the connection interface on the dissection tool involves a certain clamping force. Preferably, the pressure reservoir supplies a pressure that is higher than the pressure in the sealed collection tube and lower than the clamping force between the collection tube and the tool.

In embodiments of the method where the step of generating an overpressure involves generating a pressure of greater than atmospheric pressure, the method suitably comprises a subsequent step of allowing the collection tube to equilibrate to atmospheric pressure. This can be achieved via controlled leakage of the pressure reservoir, or by switching the valve to a third position in which the internal passageway is in fluid communication with the atmosphere or other suitable method. The collection tube can then be removed from the dissection tool.

Suitably, the method comprises controlling the airflow during the step of dissection. **In** a further development, the method comprises a step of measuring pressure or measuring airflow downstream from the filter element of the dissection tool or a step of estimating an area of biological material that has been dissected from the glass slide. The airflow is controlled by adjusting the restriction of a variable restrictor in response to the measured pressure or measured airflow or estimated area of dissected material. The advantage of the further development will be described later with reference to the apparatus of the invention.

In a further aspect, the invention defines a dissection tool for use in the method, which is disposable and can be manufactured in a straightforward and inexpensive manner. The tool has first and second ends, an internal passageway extending through a body of the tool and an air-permeable filter element arranged in the internal passageway. The tool first end is provided with an orifice and a scraping blade. The scraping blade is preferably made of a metal having a high yield stress. The tool body is preferably made of polymer material. The second end of the tool is provided with a connection interface for releasably connecting the dissection tool in an airtight manner to a mating interface on a tool carrier.

According to the invention, the tool body comprises a monolithic main body part, i.e. formed as a single piece. The filter element is fixedly retained within the main body part and the mechanical interface comprises one or more conical recesses or one or more conical protrusions formed in the main body part.

The tool is intended to be disposed of after use. It is therefore beneficial for the tool to be as inexpensive as possible, which is at least partly achieved via a straightforward manufacturing process. The method of the invention enables the dissected material to be removed from the interior of the tool without any need for dismantling of the tool The body part which retains the filter element may thus incorporate the additional functionality of the mechanical interface and may be executed as a single piece, thereby minimising the number of different parts that need to be assembled in order to manufacture the tool.

In one example, the main tool body part also incorporates the scraping blade and the entire tool body is a monolithic part. In a preferred embodiment, which allows enhanced manufacturing accuracy, the scraping blade is provided in a second, "blade" part, which is joined to the main body part. Suitably, the blade part is irreversibly coupled to the main body part, i.e. the two parts cannot be disconnected without breaking the coupling. This may be achieved via a form fit, adhesive bonding or other suitable joining method. The irreversible coupling facilitates robustness of the tool body as a whole. A dissection tool according to the invention may thus comprise a maximum of two body parts.

Preferably, the main tool body part and the blade part, when present, are formed in a moulding process from a suitable polymer material. The metal scraping blade may be embedded, at one side of the tool orifice, or may be attached via adhesive bonding or other suitable joining method. In other examples, the blade part or the entire tool body is overmoulded to the scraping blade. The moulded material can be an injection moulding grade thermoplastic polymer. The grade can be a glass- ceramic-, or carbon-filled material. The polymer can be from the class of polypropylene, polyester, polycarbonate, ABS, or other engineering thermoplastic material and blends of those that are commonly used in engineering and high precision applications

The connection interface for attaching the tool to the tool carrier on a dissection apparatus is integrally formed in the monolithic main tool body part. In order to perform high-precision dissection, it is important for the tool to be attached with precise alignment, which is achieved via a conical interconnection. In some examples, an upper end of the main tool body part comprises a number of conical pins that engage in corresponding conical recesses provided in the tool carrier. Alternatively, the tool may comprise a number of conical recesses which are adapted to engage with corresponding conical pins on the tool carrier.

In a preferred embodiment, the tool main body part comprises a single conical recess which forms part of the internal passageway.

In addition, the second end of the tool may comprise a click fitting or snap fit elements for establishing a quick coupling with the tool carrier. The aforementioned quick coupling elements are preferably also formed in the main body part of the dissection tool. The advantage of a quick coupling is that automated attachment and detachment of the tool, after dissection, and transfer are facilitated.

The tool further comprises a second connection interface, for establishing a seal with the collection tube when it is arranged around the tool orifice. In one example, the second connection interface comprises a collar with an outer diameter that is dimensioned to mate with the internal diameter of a collection tube. The collar may comprise a larger-diameter flange part, an underside of which is used contacting an upper rim of the collection tube when it is placed around the orifice. The seal may then be formed between the upper rim and the aforementioned underside, whereby the collection tube is pressed against the flange part with a certain clamping force to achieve an airtight connection. Suitably, the underside of the flange part may be provided with a layer of relatively soft material to enhance the seal. The collar may also have a conical outer surface that engages with the inner rim of the collection tube in an airtight manner.

Advantageously, for ease of manufacture, the second connection interface is integrally formed with the tool body. It may also be formed by a separate collar made of elastomeric material that is mounted around the tool body. Examples of suitable materials include rubbers (cross-linked, olefins, urethanes or silicones) and thermoplastic elastomers, such as polyesters, polyurethanes, polyolefins. A portion of the collar may have outer diameter that is slightly larger than the internal diameter of a standard-sized collection tube.

The tool of the invention further comprises an air-permeable filter element arranged within the internal passageway and spanning the full diameter thereof. Suitably, the filter element is made of a porous material with a pore size that does not allow passage of the dissected material, e.g. 10, 50, 100 or 200 µm, depending on the sample from which material is dissected. The filter element may have a woven or non-woven structure and is preferably made of a synthetic material such as polyethylene, polypropylene, polyvinylchloride, polyester, nylon, polyvinylidene fluoride or Teflon. The filter element may have a diameter of between 3.0 and 15.0 mm. Alternatively, the filter may be a thin substrate with an array of defined pores made by lithography, etching or laser ablation.

In a preferred embodiment, the filter element material is held in an essentially circular frame part, being a separate part that is inserted into the internal passageway of the main tool body part so as to be fixedly connected therewithin. The frame part may be overmoulded to the filter element material. In one example, the frame part has a larger diameter than the diameter of the tool internal passageway, and is radially retained via an interference fit.

In a further embodiment, the main tool body part is overmoulded to the filter element.

The filter element divides the internal passageway into first and second sections. The first section of the passageway will be defined as the section between the filter element and the tool orifice. The first section of the passageway is preferably straight and has a longitudinal centre axis L. The diameter of the passageway first section may be constant over its length or may vary. For example, the passageway may narrow towards the tool orifice. In use, the part of the tool body that surrounds the first section of the passageway serves as a suction nozzle and will be referred to as the tool nozzle.

The scraping blade is arranged at one side of the tool orifice. The scraping blade has a scraping edge of width w that makes contact with the biological material on the glass slide. In accordance with the invention, the scraping blade is curved around a blade axis and comprises a thin-walled tube section. The tube section may be a continuous thin-walled cylinder or a partial cylinder such as a semi-cylinder. An end face of the tube section serves as the scraping edge.

In some examples of a dissection tool, not in accordance with the invention, the nozzle is partly formed from a thin-walled tube section and the scraping blade forms part of the nozzle. The tube section may have a wall thickness of 30 - 100 µm and an outer diameter of 3.0 - 15 mm. The scraping edge of the tube section or the scraping edge of a flat blade can be perpendicular to the blade axis or may be angled for optimized scraping performance. In some examples, the blade has a ground, sharpened edge.

Typically, a dissection tool used in the invention is arranged such that the scraping blade engages a horizontal surface of the glass slide at an angle α thereto, which will be defined as a scraping angle. In some embodiments, not in accordance with the invention, the scraping blade extends in a direction parallel to the longitudinal axis L of the nozzle and the scraping angle is defined by the orientation of the nozzle axis relative to the slide surface.

In other embodiments, in accordance with the invention, where the scraping blade extends from an end face of the nozzle, the scraping blade has a blade axis that extends at an angle relative to the longitudinal axis L. The nozzle may thus be arranged such that the longitudinal axis L is perpendicular to the slide surface, and the scraping angle is defined by the angle between the slide surface and the blade axis. As will be understood, the scraping angle α can be varied by adjusting the angular orientation of the nozzle axis.

In a further development, the scraping blade of the dissection tool is provided with an anti-stick coating such as NiF or perfluoralkane, to prevent sticking of the dissected material and to ensure that it is suctioned to the underside of the filter element.

In a still further development, the nozzle comprises a nozzle extension that at least partially surrounds the tool orifice at a side opposite from the scraping blade. In an example, the tool is adapted to be arranged with a predefined scraping angle α relative to the horizontal slide surface. When the blade is in contact with the slide, a peripheral edge of the nozzle extension may be adapted to create a constant gap to the slide surface. In further examples, the peripheral edge of the nozzle extension has a predetermined profile, such that the gap varies along the circumferential direction.

The advantage of the nozzle extension is to optimize the airstream that is generated during suction, at the location of the scraping edge, thereby ensuring that all dissected material is suctioned into the body of the tool.

A dissection tool in accordance with the invention thus performs three functions: dissection, collection and transfer. Moreover, since the tool is intended to be disposed of after use, the risk of cross-contamination of the biological material is further reduced.

In a further aspect, the invention relates to an apparatus for dissecting and transferring biological material from a sample disposed on a planar substrate, which is configured to perform embodiments of the method of the invention as described above. The apparatus comprises:
- a tool carrier having a mechanical interface for establishing an airtight connection with the corresponding mechanical interface on a dissection tool, as defined in claim 1, which comprises an internal passageway extending between first and second ends of the tool, a filter element arranged within a body of the dissection tool to span the internal passageway and a scraping blade arranged at an orifice of the tool, at the tool first end;
- a platform for supporting the planar substrate;
- a stage for holding at least one collection tube;
- a positioning system configured to move the tool carrier and the platform relative to each other and control their relative positions such that the scraping blade of the dissection tool selectively engages with the biological material in a predefined region of the sample, and further configured to move the tool carrier relative to the stage such that collection tube is arranged around the tool orifice;
- a vacuum generator or fitting for connection of an external vacuum generator;
- a pressure reservoir or fitting for connection of an external pressure reservoir; and
- a valve operable between a first position in which the internal passageway of the dissection tool is in communication with the vacuum generator and a second position in which the passageway is in communication with the pressure reservoir, for causing the collected biological material to be expelled into the collection tube.

In one example, the pressure reservoir is simply the atmosphere. In a further example the pressure reservoir is a pump. In a still further example, the pressure reservoir is a vessel containing pressurized air, held at pressure of 100 - 400 kPa.

Preferably, the apparatus comprises an imaging system for obtaining an image of the biological sample. Suitably, the imaging system is configured to identify the boundary between a region of interest containing biological material to be tested and an unwanted area containing material not to be tested. In other words, the imaging system identifies the shape of the region that is to be dissected. In some examples, the biological sample is a stained tissue sample and the imaging system simply recognizes the stained region. In other examples, the imaging system may be programmed with software to process a captured image of the tissue sample and identify the region of interest based on e.g. cell structure. The system may also be configured to identify the shape of the region to be dissected by comparing the captured image with a reference image that has been marked by a pathologist. Suitably, the imaging system is configured to transfer coordinates of the identified boundary to a controller of the positioning system.

The scraping blade of the dissection tool has a scraping edge of width w. As a result of the relative movement between the blade and the planar substrate in a direction of translation, the scraping edge cuts a track through the biological sample having a corresponding width. When the blade is formed by a section of a thin-walled tube, the width of the track depends on the outer diameter of the tube and the applied contact force.

During dissection, the dissection tool may be arranged such that the scraping blade is arranged at a scraping angle of between 30 and 60 degrees, although other angles may be desirable depending on the nature of the biological sample being dissected.

In a further development, the tool carrier is pivotably mounted to the apparatus to enable adjustment of the scraping angle and, if necessary, to bring the dissection tool to a vertical position, to facilitate automated coupling/uncoupling of the dissection tool and/ or automated attachment of the collection tube around e.g. the sealing collar of the tool.

Suitably, the positioning system comprises motorized actuators for relative movement in transverse X and Y directions and in the vertical direction Z. In some embodiments, the positioning system further comprises a rotation stage for adjusting a position of the platform (and biological sample) relative to the scraping blade about a rotation axis that is perpendicular to the planar substrate.

During dissection, the dissection tool is moved relative to the platform in a direction of translation T. In a further development, the tool carrier of the apparatus is rotational about an axis normal to the platform, to enable angular adjustment of the scraping edge relative to the direction of translation. This is advantageous as it allows the effective width of the scraping edge i.e. width measured perpendicular to the direction of translation, to be varied. Suitably, the positioning system comprises an actuator for controllable adjustment of angular orientation.

The actuators of the positioning system may be coupled to the platform, for moving the biological sample relative to the dissection tool and/or the actuators may be coupled to the tool carrier for moving the dissection tool relative to the platform.

In a further development, the apparatus is equipped with an air ionizer. Contact between the scraping edge and the planar surface on which the biological material is disposed produces friction, which can lead to a build-up of static charge on the biological material. The air ionizer conditions the air flowing around the dissection tool, and thus reduces any build-up of static charge that could adversely affect the collection of biological material during dissection.

In a still further development, the apparatus is equipped with a variable restrictor arranged in line between the vacuum generator and the filter element of the dissection tool, for regulating airflow through the tool nozzle. In use of the tool, the dissected material collected at the underside of the filter element will restrict the airflow through the filter, thereby increasing the restriction of the system as a whole. This will result in a loss of pressure and a reduction in airflow that could adversely affect the suction capability of the tool nozzle. Suitably, the apparatus is adapted to reduce the restriction of the variable restrictor in response to accumulation of dissected material at the underside of the filter, to enable a constant airflow through the internal passageway and the nozzle.

In one example, the apparatus is further equipped with a pressure sensor or flow sensor arranged in line between the vacuum generator and the filter element and the variable restrictor is controlled based on the measured pressure or measured flow. In a further example, the apparatus is configured to estimate an area of biological material that has been dissected by the scraping blade, and to reduce the restriction of the variable restrictor as a function of the dissected area. The imaging system may be configured to estimate the dissected area or it may be calculated based on the width of the scraping blade and the distance of each translation movement of the blade relative to the slide.

It will be appreciated by those skilled in the art that two or more of the above-mentioned embodiments, implementations, and/or aspects of the invention may be combined in any way deemed useful.

### BRIEF DESCRIPTION OF THE DRAWINGS

The invention will now be further elucidated with reference to the embodiments described hereinafter. In the drawings,
- Fig. 1a: schematically shows an example of an apparatus equipped with a scraping blade and a vacuum generator for dissecting and transferring biological material from a glass slide to a collection tube;
- Fig. 1b: shows parts of the apparatus of Fig. 1a in a configuration in which material is removed from the slide and suctioned into a dissection tool;
- Fig. 1c: shows parts of the apparatus of Fig. 1a in a configuration in which the suctioned material is prepared for transfer to the collection tube;
- Fig. 1d: shows parts of the apparatus of Fig. 1a in a configuration in which the suctioned material is transferred to the collection tube.
- Fig. 2: is a side view of an example of a dissection tool that employs.
- Fig. 3: is a side view of an example of a dissection tool according to the invention, with key internal features indicated via dotted lines.
- Fig. 4a: is a cut side view of a further example of a dissection tool according to the invention;
- Fig. 4b: is a cross-sectional view of a filter element mounted in the tool of Fig. 4a.

It should be noted that items which have the same reference numbers in different figures, have the same structural features and the same functions, or are the same signals. Where the function and/or structure of such an item has been explained, there is no necessity for repeated explanation thereof in the detailed description.

### DETAILED DESCRIPTION OF EMBODIMENTS

Pathology diagnostic investigation of biological material, such as tissue and cells, forms the basis for many treatment decisions, particularly in oncology. For example, genomic-based tests are performed in order to inform therapy selection for individual patients diagnosed with cancer. The biological material/tissue may be obtained from a biopsy and is then, for example, embedded in paraffin and cut into thin slices which are fixed onto glass slides. These thin slices will be referred to as tissue samples. Other methods of obtaining and preparing biological material are known.

The tissue sample has a region of interest ROI containing material that is to be subjected to the diagnostic testing. The ROI can be identified by staining, or a pathologist may provide markings on a reference slide after analysis under a microscope. The ROI can also be identified via processing of a digital image of the sample. When the ROI has been identified, material is removed/dissected from the slide and then transferred to an analysis arrangement. Typically, the material is transferred to a collection tube, after which a sample preparation process steps, like cell lysis, purification and amplification, etc. and further necessary processing steps are performed. As will be understood, the reliability and efficiency of the analysis is optimized, by making sure that only dissected material from the ROI is present, but also by minimizing contamination.

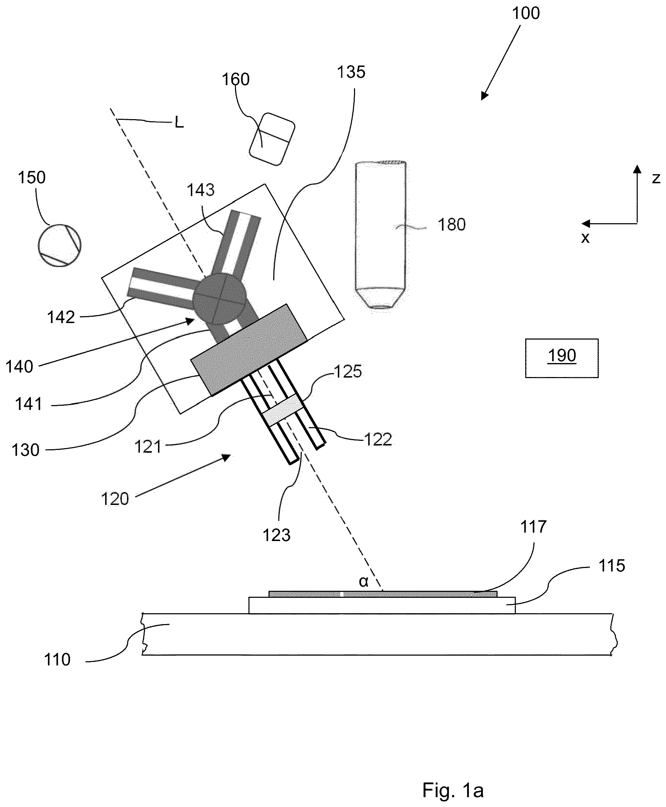

The present invention defines an automated method of dissecting biological material from a sample disposed on a planar substrate and transferring it to a collection tube in a manner that permits accurate material removal and minimizes contamination of the material to be analyzed. The method makes use of a dissection tool that is selectively connectable to a source of underpressure and a source of overpressure. The principles of a dissection tool and apparatus, which implements the method of the invention will be described with reference to Fig. 1a.

Fig 1a schematically shows an example of an apparatus 100 comprising a platform 110 for supporting a glass slide 115 on which a biological sample 117 is disposed, and a dissection tool 120 mounted to a tool carrier 130. The tool carrier is mounted to a positioning unit 135 that enables the position of the tool 120 to be adjusted relative to the platform 110. In the depicted example, the positioning unit 135 allows the tool 120 to be moved in vertical direction z and to be rotated about a vertical axis. The positioning unit forms part of a positioning system for enabling movement of the tool relative to the platform in translation directions X and Y. Preferably, the platform 110 is also movable and the positioning system comprises a controller 190 that controls relative movement such that the tool engages with the biological material 117 only in the region of interest. In the depicted example, the apparatus is further equipped with an imaging system 180 for identifying the location of the tissue and providing the ROI coordinates to the controller 190.

The dissection tool has an essentially hollow body that defines an internal passageway 121 extending between first and second ends of the tool body. An air-permeable filter element 125 is accommodated within the tool body and spans the full diameter of the internal passageway. A first section of the internal passageway is defined between the filter element 125 and an orifice 123 of the tool at the tool first end. The portion of the tool body that surrounds the first section of the internal passageway is defined as a nozzle 122, which has a longitudinal centre axis L. The nozzle axis L may coincide the with the centre axis of the tool as a whole, as is the case in the example of Fig. 1a, although this is not necessary. The second end of the dissection tool comprises a mechanical interface for releasably connecting the tool to a mating mechanical interface on the tool carrier 130.

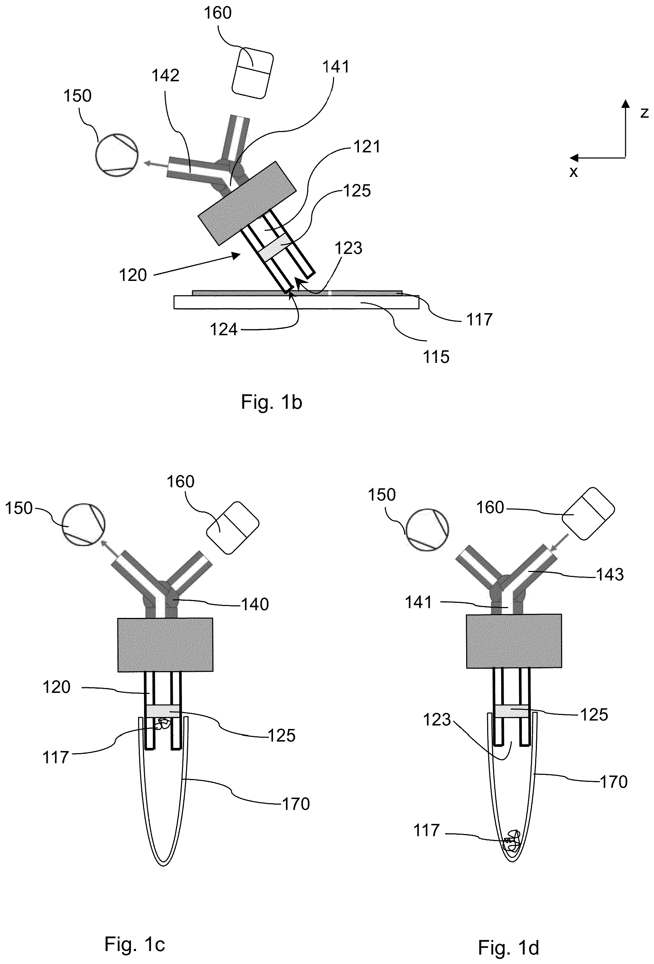

In the depicted example, the nozzle is formed by a thin-walled tube and a portion of an end face of the tube serves as a scraping blade 124 (refer Fig. 1b) that is used to mechanically detach biological material 117 disposed on the glass slide 115. The scraping blade may also be a separate part that extends from the nozzle end face at one side of the orifice 123.

During dissection, the tool is arranged such that the nozzle 122 is oriented at angle α relative to a horizontal surface of the platform 110, which also defines a scraping angle of the blade 124. The tool carrier 130 is pivotably mounted to the positioning unit 135, so as to enable adjustment of the angle α.

The apparatus is further equipped with a vacuum pump 150, a pressure reservoir 160 having a pressure of at least 100 kPa, a valve 140, and suitable tubing arranged such that the internal passageway 121 of the dissection tool 120 is selectively connectable to one of the pump 150 and the pressure reservoir 160. In the depicted example, the valve is a 3-way valve with a first port 141 in fluid communication with the mechanical interface on the tool carrier 130 and with passageway 121, a second port 142 in fluid communication with the vacuum pump 150 and a third port 143 in fluid communication with the pressure reservoir 160.

An embodiment of the method will now be described with reference to Figs. 1b - 1d, whereby for the sake of simplicity, only relevant features of the apparatus are depicted.

Fig. 1b shows the dissection tool in a material removal position and in a first step of the method, biological material 117 is scraped off the glass slide 115 and collected within the tool body. The tool 120 is arranged such that the scraping blade 124 engages with the slide surface at a scraping angle of e.g. 30 - 60 degrees. The tool is then moved relative to the slide in a translation direction x, such that a scraping edge of the blade, having a certain width in y-direction, cuts a track through the biological material 117. Suitably, the relative position of the scraping blade 124 and the slide 115 are controlled such that only material from the identified ROI is dissected via scraping.

Simultaneously with the step of scraping, an underpressure is generated at the second end of the dissection tool, which creates an uplifting airstream at the tool orifice 123, causing the dissected material to be suctioned into the passageway 121, where it is caught at an underside the filter element 125. During this step of suction, the valve 140 is in a first position in which the first and second ports 141, 142 are in fluid communication and the internal passageway 121 is in communication with the vacuum pump 150. The airstream holds the dissected material at the underside of the filter element 125 during the material removal process.

When dissection is complete, the position of the tool may be adjusted to a transfer position, in which the platform and dissection tool are distanced from each other. In a next step of the method, a collection tube 170 is attached at an outer surface of the dissection tool 120, as shown in Fig 1c, in airtight manner. Suitably, the apparatus comprises a stage for holding e.g. a tray of collection tubes and the positioning system is configured to control the movement of the tool carrier relative to the stage such that the tool 120 becomes inserted in the tube 170. The valve 140 remains in the first position and the dissected material 117 is held at the underside of the filter element 125. As a result of the continued connection with the operating vacuum pump 150, air is evacuated from the collection tube 170.

A final step of the method comprises exposing the filter element 125 to an overpressure at the second end of tool 120. This is achieved by switching the valve 140 to a second position in which the first port 141 is in fluid communication with the third port 143 and with the pressure reservoir 160, as shown in Fig. 1d. Preferably, the valve is switched to the second position when a vacuum situation is reached inside the tube 170, or a pressure essentially equal to the absolute pressure of the vacuum pump e.g. 1 - 10 kPa. Exposure to the overpressure generates a pressure pulse and an airstream, indicated by the arrow in Fig. 1d, that flows towards the tool orifice 123. The aforementioned pressure pulse catapults the dissected material 117 from the underside of the filter element 125 into the collection tube 170.

The pressure reservoir 160 can simply be the atmosphere. Preferably, to generate a pulse of sufficient magnitude to ensure that all material is ejected from the filter element 125, a pump or a reservoir of pressurized air held within a cylinder is used to supply a pressure of 200 - 400 kPa. Preferably, the supplied pressure is lower than a clamping force generated by the airtight connection of the collection tube 170 around the outer surface of the tool body. This prevents the tube from being released by the pressure pulse, which would create a risk of losing the dissected material.

After exposure to a pressure greater than 100 kPa, the pressure in the collection tube is allowed to equilibrate with the atmosphere. The collection tube may then be detached from the dissection tool 120 and is suitably covered by an end cap.

The method of the invention therefore enables automated dissection and transfer of biological material from a sample disposed on a glass slide 115 into a collection tube 170, without the need to use any liquids during dissection or to "rinse" the dissected material out of the tool body. This not only simplifies the process, but also minimizes contamination.

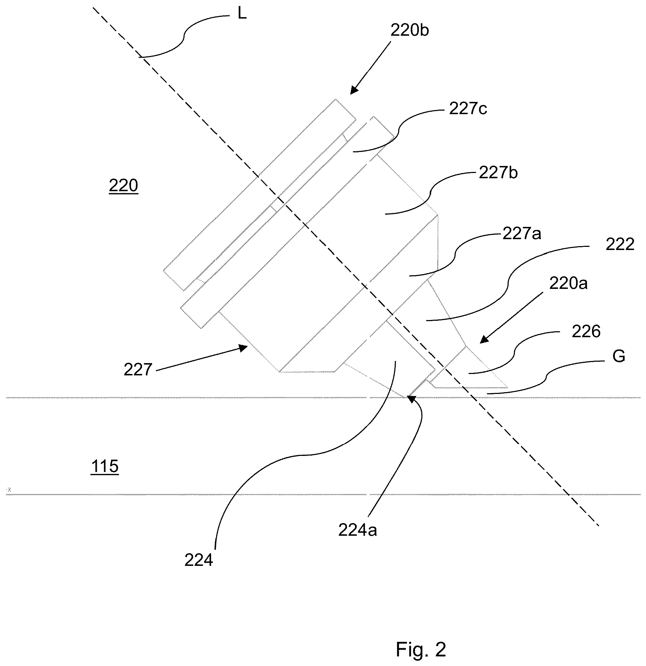

An example of a dissection tool 220, not in accordance with the invention, which suitable for use in an apparatus, is shown in Fig. 2. The tool has a hollow tool body having an internal passageway in which the filter element is arranged. A first end 220a of the tool is provided with a nozzle 222, a portion of which serves as a scraping blade 224 having a scraping edge 224a that is brought into contact with a glass slide 115 and moved relative to the slide in order to dissect material. The scraping blade 224 in this example is a separate part formed from a thin-walled tube section made of a metal with a high yield stress. Remaining parts of the nozzle 222 and tool body may be formed from polymer material that is overmoulded to the scraping blade 224. Preferably, an internal surface of the scraping blade is provided with an anti-stick coating, to prevent sticking of dissected material during scraping and during transfer.

In use of the tool, a portion of the end face of the nozzle 222 that makes contact with the biological material on the slide 115 serves as the scraping blade 224. The scraping edge 224a in the depicted example is thus arcuate in shape and has a certain width that cuts a track through the biological material when the underside of the nozzle is brought into contact with the slide surface and is moved relative to the slide. The dissected material may therefore have a ribbon shape.

As explained, the underpressure generated by the vacuum pump creates an uplifting airstream that draws in the material dissected by the scraping edge 224a. To ensure that a ribbon of dissected material is suctioned into the tool body, it is important that the lifting effect of the airstream is optimized at the location of the scraping edge. In the depicted example, this is facilitated by an extension 226 of the nozzle 222 that surrounds approximately half of the tool orifice at a circumferential side opposite from the scraping edge 224a. The extension 226 forms a curved hood, whereby a maximum length of the extension 226, measured in longitudinal direction L relative to the scraping edge 224a, occurs at a circumferential location on the nozzle of 180 degrees relative the scaping edge. At either side of this 180° position, the length of the nozzle extension reduces.

In the depicted example, the dissection tool 220 is adapted to be mounted to a tool carrier, such that the scraping blade 224 is arranged at a scraping angle of 45 degrees relative to the horizontal surface of the slide. A blade axis of the scraping blade extends parallel to a centre axis L of the nozzle and tool, meaning that the scraping angle coincides with the angle of the tool axis relative to the surface of the slide.

The scraping edge 224a forms part of a suction nozzle. A peripheral edge of the extension 226 also forms part of the nozzle and thus shortens the gap G between the nozzle and the slide surface, thereby increasing the suction force exerted on the dissected biological material. The extension 226 is designed such that the gap G is substantially constant when the tool is arranged at 45 degrees to the slide surface and the scraping edge is in contact with the slide. **In** other examples, the nozzle extension is designed such that the gap varies along the circumference.

**In** addition to improving the lifting effect at the scraping edge 224a, the extension 226 can also serve as a physical barrier to prevent sideways deflection of a dissected ribbon of material and retain it within the nozzle.

At a second end of the tool 220b, a mechanical interface is provided for releasably attaching the tool 220 at a mating interface on the tool carrier. Suitably, the interface permits an airtight connection to ensure optimal suction through the tool body and a high degree of mechanical alignment, to promote accurate positioning of the scraping edge 224a relative to the slide 115. A click-type fitting, clamping or a bayonet attachment is advantageous for the purposes of speed and automation, but other types of interface, such as threaded connection, are also possible.

The dissection tool 220 further comprises an external connection interface for receiving the collection tube that is placed over the tool body. In the depicted example, the outside of the tool body is fitted with a collar 227 made of an elastomeric sealing material for establishing an airtight connection with the collection tube. The dimensions of the collar thus depend on the collection tube used. Preferably, the collar has a chamfered edge 227a for guiding the open end of the collection tube over a main portion 227b of the collar, which has an external diameter that will seal against the internal diameter or rim of the collection tube. Suitably, the collar also has a larger-diameter flange portion 227c that will retain a rim of the collection tube, to prevent damage that might otherwise occur when the tube is attached and evacuated.

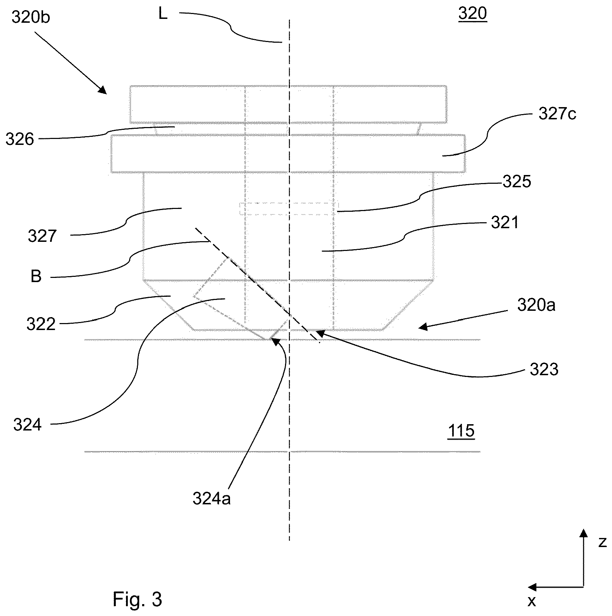

Fig. 3 shows a side view of an example of a dissection tool according to the invention. The tool 320 comprises a first, main body part 326, formed as a single piece. The internal passageway 321 extends through this body part 326 and the filter element 325 (indicated via dotted lines) is arranged in the passageway 321 and retained in the main body part. The mechanical interface for releasably connecting the tool to the carrier is provided at the tool second end 320b, in the main body part 326. The mechanical interface comprises a number of conical recesses which surround the internal passageway 321 at the tool second end 320b.

The tool further comprises a second part 327 which incorporates a nozzle part 322. The second part may be formed from polymer material that is overmoulded to the main body part 326. The scraping blade 324 is embedded in the end face of the nozzle part 322. It is also possible for the nozzle part and the main body part 326 to be entirely formed from material that is overmoulded to the metal scraping blade 324.

In the depicted example, the second part 327 not only comprises the nozzle 322 and blade 324, but further comprises the second mechanical interface or collar for connection of the collection tube. Like the example shown in Fig. 2, the collar has a flange part 327c for retaining the rim of the collection tube.

The tool 320 in this example is adapted to be arranged such the longitudinal centre axis L of the nozzle is perpendicular to the glass slide 115 during dissection. At the tool first end 320a, an end face of the nozzle 322 surrounds the orifice 323 at the entrance to the internal passageway 321, indicated via dotted lines. The metal scraping blade 324 is embedded in the nozzle, whereby part of the blade protrudes from the end face of the nozzle. The embedded part of the blade is indicated via dotted lines. In the depicted example, the scraping blade if formed from a thin piece of metal that is bent into semi-cylindrical shape around a blade axis B.

The blade axis B extends at an angle relative to the nozzle axis L, and the scraping angle is defined by the angle between the blade axis B and the slide surface. The scraping angle can, of course, be varied by adjusting the orientation of the tool body. Suitably, the scraping blade 324 extends towards the nozzle axis L, such that the scraping edge 324a of the blade is located below the tool orifice 323.

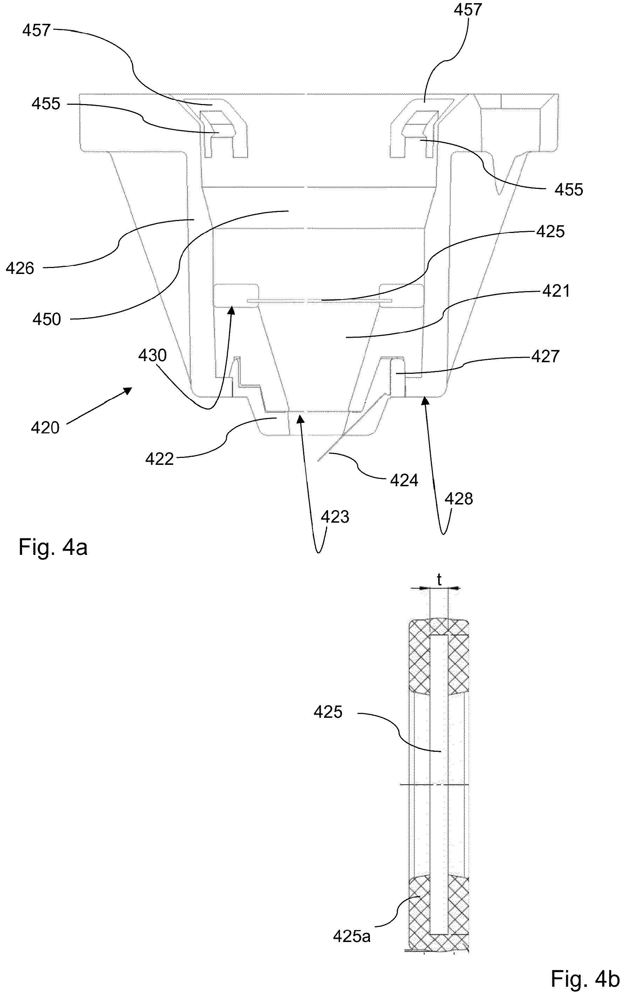

A further example of a tool according to the invention is shown in Fig. 4a in cut side view. The tool 420 comprises a main body part 426 formed from a single piece through which the tool internal passageway extends. Part of the internal passageway 421 tapers in diameter towards the tool orifice 423. A further part of the internal passageway is formed by a conical recess 450 which is adapted to connect the tool with a precise alignment to a correspondingly shaped conical protrusion on the tool carrier. As will be understood, the conical protrusion has an internal passageway which mates with the tool internal passageway. The conical recess 450 provides an airtight connection with the tool carrier protrusion. In some examples, the coupling force between the tool and the tool carrier is established via an interference fit. In the depicted example, the first connection interface additionally comprises a quick-release coupling in the form of snap-fit joints. A number of snap-fit joints 455 - three in the depicted embodiment - are provided at the entrance to the conical recess, and are adapted to engage with an annular ridge provided on the conical protrusion of the tool carrier when it is pushed into the tool recess 450. In the depicted example, the snap-fit joints are cantilever arms that extend within a corresponding indentation 457 in the tool body 426 at the entrance to the conical recess 450. A gap between each arm 455 and a radially outer wall of the indentation 457 permits flexure of the arm needed for engagement and disengagement of the tool. Suitably, the snap fit joints 455 are arranged at even angular intervals around the entrance to the conical recess 450 and are adapted to provide a stable locking force that is sufficiently large to enable precise dissection using the tool, yet small enough to permit relatively low-force disengagement.

The main tool body part 426 further comprises a seat 430 for locating the filter element 425 in axial direction. An example of a suitable filter element is shown in cross-sectional view in Fig. 4b. The filter element comprises a thin piece of porous, air-permeable woven material with a thickness t of e.g. 0.1 mm. A membrane filter may also be used, which typically has a larger thickness of e.g. 0.6 mm. The filter element 425 is held in a frame part 425a that may be moulded around an outer circumference of the filter element. The outer diameter of the frame part 425a is slightly larger than the diameter of the internal passageway, at the location of the seat 430 for the filter element 425, such that it is radially retained within the main body part 426 with an interference fit.

The scraping blade 424 of the tool is provided in a second part 427 that is joined to the main body part 426. Suitably, the second part 427 is overmoulded to the scraping blade 424 and may be irreversible coupled to the main body part 426 via a form fit, adhesive bonding or other suitable joining method. The second part 427 also comprises the tool orifice 423 and part of the nozzle 422. An outer surface of the nozzle part 422 in the second body part 427 is adapted to be inserted in a collection tube. The tool body further has a relative flat lower surface 428 for receiving the rim of the collection tube and establishing a sealing connection therewith. This sealing surface 428 can be provided on one of or both of the main body part 426 and the second part 427. Alternatively, the sealing surface may be formed by a conical outer surface of the nozzle part 422.

After dissection and tissue transfer, a dissection tool according to the invention is disposed of and a new tool is connected to the apparatus, thereby eliminating the risk of cross-contamination.

Examples, embodiments or optional features, whether indicated as non-limiting or not, are not to be understood as limiting the invention as claimed. It should be noted that the above-mentioned embodiments illustrate rather than limit the invention, and that those skilled in the art will be able to design many alternative embodiments without departing from the scope of the appended claims.

In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

### Reference numerals

100 dissection apparatus
110 platform
115 slide
117 tissue sample
120, 220, 320, 420 dissection tool
121, 321, 421 internal passageway through dissection tool
122, 222, 322, 422 nozzle
123, 323, 423 tool orifice
124, 224, 324, 424 scraping blade of dissection tool
125, 325, 425 air-permeable filter element
425a frame part of filter element
130 tool carrier
135 positioning unit
140 3-way valve
141, 142, 143 1^{st}, 2^{nd}, 3^{rd} valve ports
150 vacuum pump
160 pressure reservoir
170 collection tube
180 imaging system
190 controller of positioning system
220a, 320a 1^{st} end of dissection tool
220b, 320b 2^{nd} end of dissection tool
224a, 324a scraping edge of scraping blade
226 extension of nozzle
227, 327 sealing collar
227a chamfered edge of sealing collar
227b main body of sealing collar
227c, 327c retaining flange of sealing collar
326, 426 1^{st}, main tool body part
327, 427 2^{nd} part in which blade is incorporated
428 sealing surface of tool body
430 seat in main tool body part for locating filter element
450 conical recess for establishing airtight interface with tool carrier
455 snap fit joints
457 indentations in tool body at entrance to conical recess
L longitudinal axis of tool nozzle
B blade axis
α scraping angle of blade relative to slide surface
G gap between nozzle and slide surface

## Claims

1. Dissection tool (320, 420) for removing and collecting biological material (117) from a sample disposed on a planar substrate (115), the tool comprising a tool body through which an internal passageway (321) extends between first and second ends (320a, 320b), wherein the tool further comprises:
• a scraping blade (324, 424) arranged at an orifice (123, 323) of the tool, provided at the tool first end (320a);
• a first connection interface, provided at the tool second end (320b) for releasably connecting the dissection tool in an airtight manner to a mating interface on a tool carrier (130); and
• an air-permeable filter element (325, 425) arranged within the internal passageway (321) to span the full diameter thereof;
wherein:
- the tool body comprises a main body part (326, 426), formed as a single piece, in which the first connection interface is integrally formed, whereby the first connection interface comprises at least one conical recess (450) or at least one conical protrusion;
- the filter element (325, 425) is fixedly retained within the main body part (326. 426); and
- a first section of the internal passageway, which extends between the filter element and the tool orifice, has a longitudinal centre axis L;.
**characterized in that**:
the scraping blade is arranged at one side of the tool orifice and comprises a partly cylindrical tube section that is curved around a blade axis B, whereby the blade axis B extends at an angle towards the longitudinal centre axis L of the first section of the internal passageway.

2. Dissection tool according to claim 1, wherein the scraping blade (324, 424) is incorporated in the main tool body part (326, 426).

3. Dissection tool according to claim 1, wherein the scraping blade (324, 424) is incorporated in a second part (327, 427) that is joined to the main tool body part (326, 426).

4. Dissection tool according to any preceding claim, wherein the filter element (325, 425) is held in a separate frame part (425a) that is fixedly retained within the main tool body part (326, 426).

5. Dissection tool according to any of claims 1 - 3, wherein the main tool body part (326, 426) is overmoulded to the filter element (325, 425).

6. Dissection tool according to any preceding claim , wherein the first connection interface comprises a single conical recess (450) which forms part of the tool internal passageway.

7. Dissection tool according to any preceding claim, further comprising a second connection interface (327, 428) for establishing an airtight connection with a collection tube (170).

8. Dissection tool according to claim 7, wherein the second connection interface is integrally formed in the tool body and comprises a relatively large-diameter portion (327c) having an underside (428) adapted to receive an upper rim of the collection tube (170).

9. An automated method of transferring biological material (117) from a sample disposed on a planar substrate (115) into a collection tube (170), using a dissection tool (120, 220, 320, 420)in accordance with any of claims 1 - 8 , the method comprising steps of:
a) physically detaching the biological material from the planar substrate (115) using the scraping blade (124, 224, 324, 424);
b) during step a), generating an underpressure at the tool second end (220b, 320b), to create an uplifting airflow at the tool orifice (123, 323, 423), which suctions detached material into the internal passageway (121, 321), where it is held at an underside of the filter element (125, 325, 425);
c) arranging the collection tube (170) around the orifice, so as to be in sealing contact with a connection interface (227, 327, 428) on the dissection tool, and
d) generating an overpressure at the tool second end, so as to create an airstream and pressure pulse that ejects the biological material held at the underside of the filter element (125, 325, 425), transferring the material (117) into the collection tube (170).

10. The method of claim 9 wherein:
• the step of generating an underpressure comprises connecting the tool internal passageway (121, 321) to a vacuum generator (150);
• the step of generating an overpressure comprises connecting the internal passageway to a pressure reservoir (160); and
• the method comprises a further step of using the vacuum generator to evacuate air from the collection tube (170) before the internal passageway is connected to the pressure reservoir.

11. The method of claim 9 or 10, wherein step a) comprises bringing the scraping blade (124, 224, 324) into contact with the planar substrate (115) and moving the dissection tool relative thereto and wherein the method further comprises controlling the relative positions of the dissection tool (120, 220, 320) and the substrate such that the scraping blade engages with the biological material only in a region of interest.

12. The method of any of claims 9 - 11, wherein step b) comprises controlling an airflow through the dissection tool by changing the restriction of a variable restrictor in response to one or more of:
• a pressure or airflow, measured downstream from the filter element (125, 325, 425) of the dissection tool;
• an estimated area of biological material that has been detached from the substrate during step a).

13. The method of any of claims 9 - 12, wherein step d) comprises generating an overpressure of greater than atmospheric pressure and wherein the method comprises a further step of allowing the collection tube (170) to equilibrate to atmospheric pressure, prior to removal of the collection tube from the dissection tool (120, 220, 320, 420).

14. Apparatus for dissecting and transferring biological material from a sample disposed on a planar substrate (115) into a collection tube (170), wherein the apparatus is configured to execute the method of any of claims 9 - 13 and comprises:
• a tool carrier (130) having a mechanical interface for an airtight connection with a dissection tool (120, 220, 320, 420) in accordance with any of claims 1 - 8;
• a platform (110) for supporting the planar substrate (115);
• a stage for holding at least one collection tube (170);
• a positioning system (135) configured to move the tool carrier (130) and the platform (110) relative to each other and control their relative positions such that the scraping blade (124, 224, 324) of the dissection tool selectively engages with the biological material (117) in a predefined region thereof and further configured to move the tool carrier relative to the stage, such that the collection tube is arranged around the tool orifice in an airtight manner after dissection is complete;
• a vacuum generator (150) or a fitting for connection of an external vacuum generator;
• a pressure reservoir (160) or a fitting for connection of an external pressure reservoir;
• a valve (140) operable between a first position in which the internal passageway (121, 321) of the dissection tool is in communication with the vacuum generator (150) and a second position in which the passageway is in communication with the pressure reservoir (160), for causing the collected biological material to be expelled into the collection tube.

15. Apparatus according to claim 14, wherein the mechanical interface on the tool carrier (130) comprises a hollow conical protrusion for engaging in a corresponding conical recess (450) in the dissection tool.

## Patentansprüche

1. Dissektionswerkzeug (320, 420) zum Entfernen und Sammeln von biologischem Material (117) aus einer Probe, die auf einem planaren Substrat (115) angeordnet ist, wobei das Werkzeug einen Werkzeugkörper umfasst, durch den ein innerer Durchgang (321) zwischen einem ersten und einem zweiten Ende (320a, 320b) verläuft, wobei das Werkzeug ferner umfasst:
• eine Schaberklinge (324, 424), die an einer Öffnung (123, 323) des Werkzeugs angeordnet ist, die an dem ersten Ende (320a) des Werkzeugs bereitgestellt ist;
• eine erste Verbindungskopplung, die an dem zweiten Ende (320b) des Werkzeugs bereitgestellt ist, um das Dissektionswerkzeug auf eine luftdichte Weise lösbar mit einer passenden Kopplung an einem Werkzeugträger (130) zu verbinden; und
• ein luftdurchlässiges Filterelement (325, 425), das in dem inneren Durchgang (321) angeordnet ist, um dessen vollen Durchmesser zu überspannen;
wobei:
- der Werkzeugkörper ein als einzelnes Stück gebildetes Hauptkörperteil (326, 426) umfasst, in dem die erste Verbindungskopplung integral gebildet ist, wobei die erste Verbindungskopplung wenigstens eine konische Vertiefung (450) oder wenigstens einen konischen Vorsprung umfasst;
- das Filterelement (325, 425) fest in dem Hauptkörperteil (326, 426) gehalten wird; und
- ein erster Abschnitt des inneren Durchgangs, der zwischen dem Filterelement und der Werkzeugöffnung verläuft, eine Längsmittelachse L aufweist;
**dadurch gekennzeichnet, dass**:
die Schaberklinge an einer Seite der Werkzeugöffnung angeordnet ist und einen teilweise zylindrischen Rohrabschnitt aufweist, der um eine Klingenachse B gekrümmt ist, wobei die Klingenachse B in einem Winkel in Richtung zu der Längsmittelachse L des ersten Abschnitts des inneren Durchgangs verläuft.

2. Dissektionswerkzeug nach Anspruch 1, wobei die Schaberklinge (324, 424) in das Werkzeug-Hauptkörperteil (326, 426) integriert ist.

3. Dissektionswerkzeug nach Anspruch 1, wobei die Schaberklinge (324, 424) in ein zweites Teil (327, 427) integriert ist, das mit dem Werkzeug-Hauptkörperteil (326, 426) verbunden ist.

4. Dissektionswerkzeug nach einem der vorstehenden Ansprüche, wobei das Filterelement (325, 425) in einem getrennten Rahmenteil (425a) gehalten ist, das fest in dem Werkzeug-Hauptkörperteil (326, 426) gehalten wird.

5. Dissektionswerkzeug nach einem der Ansprüche 1-3, wobei das Werkzeug-Hauptkörperteil (326, 426) mit dem Filterelement (325, 425) umspritzt ist.

6. Dissektionswerkzeug nach einem der vorstehenden Ansprüche, wobei die erste Verbindungskopplung eine einzelne konische Vertiefung (450) umfasst, die einen Teil des inneren Durchgangs des Werkzeugs bildet.

7. Dissektionswerkzeug nach einem der vorstehenden Ansprüche, ferner umfassend eine zweite Verbindungskopplung (327, 428) zum Einrichten einer luftdichten Verbindung mit einem Sammelrohr (170).

8. Dissektionswerkzeug nach Anspruch 7, wobei die zweite Verbindungskopplung integral in dem Werkzeugkörper gebildet ist und einen Abschnitt (327c) mit vergleichsweise großem Durchmesser umfasst, der eine Unterseite (428) aufweist, die dafür ausgelegt ist, einen oberen Rand des Sammelrohrs (170) aufzunehmen.

9. Automatisiertes Verfahren zum Überführen von biologischem Material (117) von einer auf einem planaren Substrat (115) angeordneten Probe in ein Sammelrohr (170) unter Verwendung eines Dissektionswerkzeugs (120, 220, 320, 420) nach einem der Ansprüche 1-8, wobei das Verfahren die Schritte umfasst:
a) physisches Ablösen des biologischen Materials von dem planaren Substrat (115) unter Verwendung der Schaberklinge (124, 224, 324, 424);
b) bei Schritt a) Erzeugen eines Unterdrucks an dem zweiten Ende des Werkzeugs (220b, 320b), um einen hochhebenden Luftstrom an der Werkzeugöffnung (123, 323, 423) zu erzeugen, der abgetrenntes Material in den inneren Durchgang (121, 321) saugt, wo es an einer Unterseite des Filterelements (125, 325, 425) gehalten wird;
c) Anordnen des Sammelrohrs (170) um die Öffnung, so dass es abdichtenden Kontakt mit einer Verbindungskopplung (227, 327, 428) an dem Dissektionswerkzeug bildet, und
d) Erzeugen eines Überdrucks an dem zweiten Ende des Werkzeugs, um einen Luftstrom- und Druckimpuls zu erzeugen, der das an der Unterseite des Filterelements (125, 325, 425) gehaltene biologische Material ausstößt und das Material (117) in das Sammelrohr (170) überführt.

10. Verfahren nach Anspruch 9, wobei:
• der Schritt des Erzeugens eines Unterdrucks Verbinden des inneren Durchgangs (121, 321) des Werkzeugs mit einem Vakuumgenerator (150) umfasst;
• der Schritt des Erzeugens eines Überdrucks Verbinden des inneren Durchgangs mit einem Druckbehälter (160) umfasst; und
• das Verfahren einen weiteren Schritt des Verwendens des Vakuumgenerators zum Evakuieren von Luft aus dem Sammelrohr (170), bevor der interne Durchgang mit dem Druckbehälter verbunden wird, umfasst.

11. Verfahren nach Anspruch 9 oder 10, wobei Schritt a) Inkontaktbringen der Schaberklinge (124, 224, 324) mit dem planaren Substrat (115) und Bewegen des Dissektionswerkzeugs relativ dazu umfasst und wobei das Verfahren ferner Steuern der relativen Positionen des Dissektionswerkzeugs (120, 220, 320) und des Substrats umfasst, so dass die Schaberklinge nur in einem Bereich von Interesse mit dem biologischen Material in Eingriff kommt.

12. Verfahren nach einem der Ansprüche 9-11, wobei Schritt b) Steuern eines Luftstroms durch das Dissektionswerkzeug durch Verändern der Drosselung einer variablen Drossel als Reaktion auf eines oder mehrere umfasst von:
• einem Druck oder Luftstrom, gemessen stromabwärts von dem Filterelement (125, 325, 425) des Dissektionswerkzeugs;
• eine abgeschätzten Fläche von biologischem Material, das bei Schritt a) von dem Substrat abgelöst worden ist.

13. Verfahren nach einem der Ansprüche 9-12, wobei Schritt d) Erzeugen eines Überdrucks von mehr als Atmosphärendruck umfasst und wobei das Verfahren einen weiteren Schritt des Zulassens, dass das Sammelrohr (170) mit Atmosphärendruck äquilibriert, vor Entfernen des Sammelrohrs aus dem Dissektionswerkzeug (120, 220, 320, 420) umfasst.

14. Vorrichtung zum Sezieren und Überführen von biologischem Material von einer Probe, die auf einem planaren Substrat (115) angeordnet ist, in ein Sammelrohr (170), wobei die Vorrichtung dafür gestaltet ist, das Verfahren nach einem der Ansprüche 9-13 auszuführen, und umfasst:
• einen Werkzeugträger (130), der eine mechanische Kopplung für eine luftdichte Verbindung mit einem Dissektionswerkzeug (120, 220, 320, 420) nach einem der Ansprüche 1-8 aufweist;
• eine Plattform (110) zum Tragen des planaren Substrats (115);
• eine Bühne zum Halten wenigstens eines Sammelrohrs (170);
• ein Positioniersystem (135), dafür gestaltet, den Werkzeugträger (130) und die Plattform (110) relativ zueinander zu bewegen und ihre relativen Positionen so zu steuern, dass die Schaberklinge (124, 224, 324) des Dissektionswerkzeugs selektiv mit dem biologischen Material (117) in einem vordefinierten Bereich davon in Eingriff kommt, und ferner dafür getsaltet ist, den Werkzeugträger relativ zu der Bühne zu bewegen, so dass das Sammelrohr um die Werkzeugöffnung auf eine luftdichte Weise angeordnet ist, nachdem die Dissektion abgeschlossen ist;
• einen Vakuumgenerator (150) oder ein Formstück zum Anschluss eines externen Vakuumgenerators;
• einen Druckbehälter (160) oder ein Formstück um Anschluss eines externen Druckbehälters;
• ein Ventil (140), das zwischen einer ersten Position, in der der innere Durchgang (121, 321) des Dissektionswerkzeugs in Verbindung mit dem Vakuumgenerator (150) steht, und einer zweiten Position, in der der Durchgang in Verbindung mit dem Druckbehälter (160) steht, betreibbar ist, um zu bewirken, dass das gesammelte biologische Material in das Sammelrohr ausgestoßen wird.

15. Vorrichtung nach Anspruch 14, wobei die mechanische Schnittstelle an dem Werkzeugträger (130) einen hohlen konischen Vorsprung zum Eingriff in eine entsprechende konische Vertiefung (450) in dem Dissektionswerkzeug umfasst.

## Revendications

1. Outil de dissection (320, 420) pour retirer et recueillir un matériel biologique (117) d'un échantillon disposé sur un substrat plan (115), l'outil comprenant un corps d'outil à travers lequel un passage interne (321) s'étend entre des première et seconde extrémités (320a, 320b), l'outil comprenant en outre :
• une lame de raclage (324, 424) agencée au niveau d'un orifice (123, 323) de l'outil, prévu au niveau de la première extrémité (320a) ;
• une première interface de raccordement, prévue au niveau de la seconde extrémité de l'outil (320b) pour raccorder de manière étanche l'outil de dissection à une interface de conjugaison sur un porte-outil (130) ; et
• un élément filtrant (325, 425) perméable à l'air agencé au sein du passage interne (321) pour couvrir tout son diamètre ;
dans lequel :
- le corps d'outil comprenant une partie de corps principale (326, 426), formée d'une seule pièce, dans laquelle la première interface de raccordement est formée d'un seul tenant, ladite première interface de raccordement comprenant au moins un évidement conique (450) ou au moins une saillie conique ;
- l'élément filtrant (325, 425) étant retenu de manière fixe à l'intérieur de la partie de corps principale (326, 426) ; et
- une première section du passage interne, qui s'étend entre l'élément filtrant et l'orifice de l'outil, présentant un axe central longitudinal L ;
**caractérisé en ce que** :
la lame de raclage est agencée sur un côté de l'orifice de l'outil et comprend une section de tube partiellement cylindrique qui est courbée autour d'un axe de lame B, l'axe de lame B s'étendant selon un angle vers l'axe central longitudinal L de la première section du passage interne.

2. Outil de dissection selon la revendication 1, la lame de raclage (324, 424) étant incorporée dans la partie principale de corps d'outil (326, 426).

3. Outil de dissection selon la revendication 1, la lame de raclage (324, 424) étant incorporée dans une seconde partie (327, 427) qui est jointe à la partie principale de corps d'outil (326, 426).

4. Outil de dissection selon l'une quelconque des revendications précédentes, l'élément filtrant (325, 425) étant maintenu dans une partie de cadre séparée (425a) qui est retenue de manière fixe à l'intérieur de la partie principale de corps d'outil (326, 426).

5. Outil de dissection selon l'une quelconque des revendications 1 à 3, la partie principale de corps d'outil (326, 426) étant surmoulée sur l'élément filtrant (325, 425).

6. Outil de dissection selon l'une quelconque des revendications précédentes, la première interface de raccordement comprenant un évidement conique unique (450) qui fait partie du passage interne de l'outil.

7. Outil de dissection selon l'une quelconque des revendications précédentes, comprenant en outre une seconde interface de raccordement (327, 428) pour établir un raccordement étanche à l'air avec un tube de collecte (170).

8. Outil de dissection selon la revendication 7, la seconde interface de raccordement étant formée d'un seul tenant dans le corps d'outil et comprenant une portion de diamètre relativement large (327c) ayant une face inférieure (428) adaptée pour recevoir un bord supérieur du tube de collecte (170).

9. Procédé automatisé de transfert de matériel biologique (117) à partir d'un échantillon disposé sur un substrat plan (115) dans un tube de collecte (170), utilisant un outil de dissection (120, 220, 320, 420) selon l'une quelconque des revendications 1 à 8, le procédé comprenant les étapes de :
a) détachement physique du matériel biologique du substrat plan (115) au moyen de la lame de raclage (124, 224, 324, 424) ;
b) pendant l'étape a), génération d'une sous-pression au niveau de la seconde extrémité de l'outil (220b, 320b) pour créer un flux d'air ascendant au niveau de l'orifice de l'outil (123, 323, 423), lequel aspire le matériel détaché dans le passage interne (121, 321) où il est maintenu contre une face inférieure de l'élément filtrant (125, 325, 425) ;
c) agencement du tube de collecte (170) autour de l'orifice de manière à être en contact étanche avec une interface de raccordement (227, 327, 428) sur l'outil de dissection ; et
d) génération d'une surpression au niveau de la seconde extrémité de l'outil de manière à créer un flux d'air et une impulsion de pression qui éjecte le matériel biologique maintenu contre la face inférieure de l'élément filtrant (125, 325, 425), transférant le matériel (117) dans le tube de collecte (170).

10. Procédé selon la revendication 9,
• l'étape de génération d'une sous-pression comprenant le raccordement du passage interne de l'outil (121, 321) à un générateur de vide (150) ;
• l'étape de génération d'une surpression comprenant le raccordement du passage interne à un réservoir de pression (160) ; et
• le procédé comprenant une étape supplémentaire consistant à utiliser le générateur de vide pour évacuer l'air du tube de collecte (170) avant que le passage interne ne soit raccordé au réservoir de pression.

11. Procédé selon la revendication 9 ou 10, l'étape a) comprenant la mise en contact de la lame de raclage (124, 224, 324) avec le substrat plan (115) et le déplacement de l'outil de dissection par rapport à celui-ci, et le procédé comprenant en outre la commande des positions relatives de l'outil de dissection (120, 220, 320) et du substrat de telle sorte que la lame de raclage s'engage avec le matériel biologique uniquement dans une région d'intérêt.

12. Procédé selon l'une quelconque des revendications 9 à 11, l'étape b) comprenant la commande d'un flux d'air à travers l'outil de dissection en changeant la restriction d'un restricteur variable en réponse à une ou plusieurs parmi :
• une pression ou un flux d'air, mesuré en aval de l'élément filtrant (125, 325, 425) de l'outil de dissection ;
• une surface estimée de matériel biologique qui a été détachée du substrat lors de l'étape a).

13. Procédé selon l'une quelconque des revendications 9 à 12, l'étape d) comprenant la génération d'une surpression supérieure à la pression atmosphérique, et le procédé comprenant une étape supplémentaire pour permettre au tube de collecte (170) de s'équilibrer à la pression atmosphérique avant le retrait du tube de collecte de l'outil de dissection (120, 220, 320, 420).

14. Appareil pour disséquer et transférer du matériel biologique à partir d'un échantillon disposé sur un substrat plan (115) dans un tube de collecte (170), l'appareil étant configuré pour exécuter le procédé selon l'une quelconque des revendications 9 à 13 et comprenant :
• un porte-outil (130) ayant une interface mécanique pour un raccordement étanche à l'air avec un outil de dissection (120, 220, 320, 420) selon l'une quelconque des revendications 1 à 8 ;
• une plateforme (110) pour supporter le substrat plan (115) ;
• un support pour maintenir au moins un tube de collecte (170) ;
• un système de positionnement (135) configuré pour déplacer le porte-outil (130) et la plateforme (110) l'un par rapport à l'autre et commander leurs positions relatives de sorte que la lame de raclage (124, 224, 324) de l'outil de dissection s'engage sélectivement avec le matériel biologique (117) dans une région prédéfinie de celui-ci et en outre configuré pour déplacer le porte-outil par rapport au support de sorte que le tube de collecte est agencé autour de l'orifice de l'outil de manière étanche après que la dissection est terminée ;
• un générateur de vide (150) ou un raccord pour le raccordement d'un générateur de vide externe ;
• un réservoir de pression (160) ou un raccord pour le raccordement d'un réservoir de pression externe ;
• une vanne (140) actionnable entre une première position dans laquelle le passage interne (121, 321) de l'outil de dissection est en communication avec le générateur de vide (150) et une seconde position dans laquelle le passage est en communication avec le réservoir de pression (160) pour provoquer l'expulsion du matériel biologique collecté dans le tube de collecte.

15. Appareil selon la revendication 14, l'interface mécanique sur le porte-outil (130) comprenant une saillie conique creuse pour s'engager dans l'au moins un évidement conique correspondant (450) dans l'outil de dissection.
